(19) Europäisches Patentamt
European Patent Office
Office européen des brevets

(11) Publication number: **0 160 564 B1**

(12) **EUROPEAN PATENT SPECIFICATION**

(45) Date of publication of patent specification: **31.08.94** (51) Int. Cl.[5]: **C07D 501/46**

(21) Application number: **85303031.0**

(22) Date of filing: **29.04.85**

The file contains technical information submitted after the application was filed and not included in this specification

(54) **Process for preparing cephalosporin compounds.**

(30) Priority: **30.04.84 GB 8410992**

(43) Date of publication of application:
**06.11.85 Bulletin 85/45**

(45) Publication of the grant of the patent:
**31.08.94 Bulletin 94/35**

(84) Designated Contracting States:
**AT BE CH DE FR GB IT LI NL SE**

(56) References cited:
**EP-A- 0 075 805**
**FR-A- 2 421 907**
**GB-A- 1 571 683**
**GB-A- 2 025 398**
**US-A- 3 971 778**

(73) Proprietor: **GLAXO GROUP LIMITED**
**Glaxo House,**
**Berkeley Avenue**
**Greenford, Middlesex UB6 0NN (GB)**

(72) Inventor: **Looker, Brian Edgar**
**28 Middleton Avenue**
**Greenford Middlesex (GB)**

(74) Representative: **Pett, Christopher Phineas et al**
**Frank B.Dehn & Co.**
**Imperial House**
**15-19 Kingsway**
**London WC2B 6UZ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 160 564 B1

## Description

This invention relates to improvements in or relating to cephalosporins. More particularly it relates to processes for the preparation of the cephalosporin antibiotic ceftazidime.

Ceftazidime ((6R,7R)-7-[(Z)-2-(2-aminothiazol-4-yl)-2-(2-carboxyprop-2-oxyimino)acetamido]-3-(1-pyridiniummethyl)ceph-3-em-4-carboyylate) is a valuable antibiotic having broad spectrum activity, in particular, unusually high activity against gram-negative organisms, including Pseudomonas organisms, as described in UK Patent Specification No. 2025398.

Semi-synthetic cephalosporins may conventionally be prepared by the acylation of an appropriate 7-aminocephalosporin with a compound serving to introduce a preformed 7-substituent, or by modification at the 3-position, for example the reaction of a 3-acyloxymethyl-or 3-halomethylcephalosporin with a nucleophile, to introduce the desired 3-substituent. These general processes are described in U.K. Patent No. 2025398 for the preparation of ceftazidime.

We have now devised a process for the preparation of ceftazidime, and salts and protected derivatives thereof in which the 2-carboxyprop-2-yl moiety in the 7-substituent of ceftazidime is introduced as the last major chemical step in the synthesis, by an etherification reaction.

According to one aspect of the invention, therefore, we provide a process for the preparation of compounds of general formula (I)

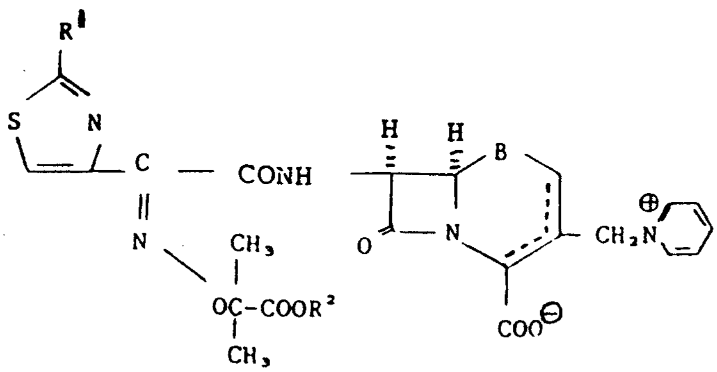

(I)

(wherein $R^1$ represents an amino or protected amino group, $R^2$ is hydrogen or a carboxyl blocking group, B is $>S$ or $>S \rightarrow O$ and the dotted line indicates that the compound is a ceph-2-em or ceph-3-em compound) or a corresponding compound having at the 4-position a group of formula $-COOR^3$ (where $R^3$ is a carboxyl blocking group) and having an associated anion $A^{\ominus}$, and salts and esters thereof which comprises reacting a compound of formula (II)

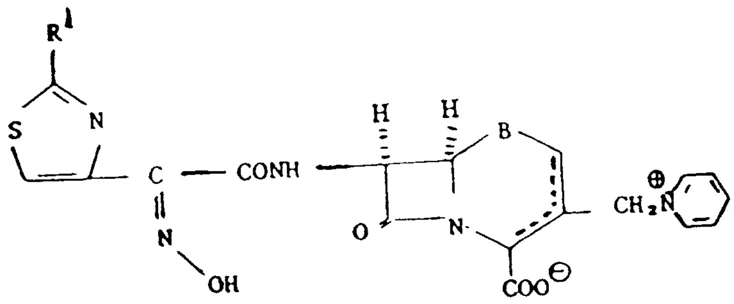

(II)

(wherein $R^1$, B and the dotted line are as defined above) or a corresponding compound having a group of formula $COOR^3$ at the 4-position (wherein $R^3$ is as defined above) together with an associated anion $A^{\ominus}$, or a salt thereof, with an agent serving to introduce the etherifying group

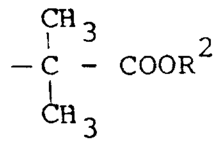

Such an agent being a compound of formula (III)

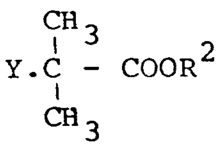 (III)

(where Y is a leaving group, in particular a halogen e.g. chloro, bromo or iodo; sulphate; or a hydrocarbylsulphonyloxy, e.g. methanesulphonyloxy or p-toluenesulphonyloxy group, and $R^2$ is as defined above) in the presence of a base selected from an alkali metal carbonate (e.g. sodium, potassium or lithium carbonate), hydride (e.g. sodium, potassium or lithium t-butoxide), dialkylamide (e.g. lithium diisopropylamide) or a disilylamide.

The anion $A^{\ominus}$ which may be associated with a compound of formula (I) or (II) may be derived from an organic or inorganic acid, such as an alkyl-, aryl- or aralkyl- carboxylic or -sulphonic acid or a mineral acid. Examples of such acids include formic, trifluoroacetic, methanesulphonic, p-toluene-sulphonic, hydrochloric, hydrochloric, hydrobromic, phosphoric and sulphuric acids.

The etherification reaction may be effected in an organic medium, conveniently at a temperature in the range -50 to +100 °C, preferably 0-50 °C.

Examples of reaction media which may be employed include ethers, e.g. cyclic ethers such as tetrahydrofuran or dioxan or acylic ethers such as diethyl ether or diglyme; amides such as N,N-dimethylformamide, N,N-dimethylacetamide or hexamethylphosphoramide; lower ketones such as acetone; nitriles such as acetonitrile; nitroalkanes such as nitromethane; sulphoxides such as dimethylsulphoxide; sulphones such as sulpholane; hydrocarbons, such as benzene or hexane; and esters such as ethyl acetate, as well as mixtures of such solvents. The reaction medium may contain some water, but is preferably anhydrous.

The etherification is carried out in the presence of an agent serving to generate an anion from the hydroxime function, and/or as an acid binding agent, i.e. a base selected from the group listed above. If the compound of formula (II) is employed in the form of an acid addition salt, the base should be present in sufficient quantity to neutralize rapidly the acid in question.

The etherification reaction may be effected in a single liquid-phase or a two liquid phase reaction medium. When a two-phase system is used the reaction is generally carried out in the presence of the base e.g. an alkali metal carbonate or an alkali metal hydroxide together with a phase transfer reagent, such as a crown ether (i.e. a macrocyclic polyether). The crown ether may contain for example a number of ethyleneoxy units, which may be substituted, e.g. by phenyl or cyclohexyl groups joined to form a ring. Examples of such a reagent include 18-crown-6 (containing 6 ethyleneoxy units) e.g. dibenzo-or dicyclohexyl 18-crown-6, and 15-crown-5 (containing 5-ethyleneoxy units). A preferred two-phase medium is water/dichloromethane.

The etherification reaction may be followed where necessary and/or desired by conversion of the compound of formula (I) initially obtained into a different compound of formula (I), for example by means of one or more of the following reactions:-

(i) reduction of a compound wherein B is $>$S→O to a compound wherein B is $>$S;

(ii) conversion of a $\Delta^2$-isomer into a $\Delta^3$-isomer;

(iii) removal of any carboxyl-blocking or amino protecting groups; and

(iv) formation of a non-toxic salt or a non-toxic metabolically labile ester.

These reactions may be effected by conventional methods, and in any convenient order.

Thus, if desired, a $\Delta^2$- cephalosporin obtained in accordance with the process of the invention may be converted into the corresponding $\Delta^3$- derivative by, for example, treatment of the $\Delta^2$- ester with a base, such as pyridine or triethylamine.

A ceph-2-em reaction product may also be oxidised to yield the corresponding ceph-3-em 1-oxide, for example by reaction with a peracid, e.g. peracetic or m-chloroperbenzoic acid; the resulting sulphoxide may subsequently be reduced as described hereinafter to yield the corresponding ceph-3-em sulphide.

Where a compound of formula (I) is obtained in which B is >S→O this may, if desired, be converted into the corresponding sulphide by, for example, reduction of the corresponding acyloxysulphonium or alkoxysulphonium salt prepared in situ by reaction with e.g. acetyl chloride in the case of an acetoxysulphonium salt, reduction being effected by, for example, sodium dithionite or by iodide ion as in a solution of potassium iodide in a solvent e.g. acetic acid, acetone, tetrahydrofuran, dioxan, dimethylformamide or dimethylacetamide. The reaction may be effected at a temperature of from -20° to +50°C.

Carboxyl blocking groups which may be present in the compounds of general formula (I) or any of the intermediates described herein may be any of the conventional carboxyl protecting groups known in the art, a list of representative protected carboxyl groups being included in British Patent No. 1399086. The carboxyl blocking group may be for example the residue of an ester-forming aliphatic or araliphatic alcohol or an ester-forming phenol, silanol or stannanol preferably containing 1-20 carbon atoms. Thus, carboxyl blocking groups which may conveniently be employed include aryl lower alkyl groups, such as p-methoxybenzyl, p-nitrobenzyl and diphenylmethyl; lower alkyl groups such as t-butyl; lower haloalkyl groups such as 2,2,2-trichloroethyl; and tri(lower alkyl) silyl groups such as trimethylsilyl.

An amino protecting group present in the compounds of formula (I) or intermediates therefor is conveniently a group which may be readily removed at an appropriate stage in the reaction sequence, for example an aralkyl group, such as triphenylmethyl, or an acyl group, such as formyl, t-butoxycarbonyl, 2,2,2-trichloroethoxycarbonyl, isoamyloxycarbonyl or chloroacetyl.

Any carboxyl blocking or amino protecting group present in the compound of formula (I) may be removed, if desired, by any appropriate methods known in the art. Suitable methods for removing carboxyl protecting groups include acid or base hydrolysis and reduction. Thus, for example, diphenylmethyl and t-butyl carboxyl blocking groups may conveniently be removed by acid hydrolysis. A p-nitrobenzyl group may conveniently be cleaved by reduction, effected for example by zinc and acetic acid, or sodium dithionite or iodide ion in water conveniently in the presence of a water-miscible solvent such as acetone, tetrahydrofuran, dioxan, dimethylformamide or dimethylacetamide.

Methods of removing amino protecting groups are also well known in the art. A trityl group may for example be removed using an optionally halogenated carboxylic acid e.g. acetic acid, formic acid, chloroacetic acid or trifluoroacetic acid or using a mineral acid, e.g. hydrochloric acid or mixtures of such acids, preferably in the presence of a protic solvent such as water. Acid-labile acyl amino-protecting groups, e.g. formyl, t-butoxycarbonyl or isoamyloxycarbonyl may also be removed, where appropriate under the conditions described above for a trityl group. Other acyl amino-protecting groups e.g. 2,2,2-trichloroethoxycarbonyl may be removed, for example, by reduction.

It will be appreciated that the use of amino protecting and carboxyl blocking groups is well known in the art. Relevant examples of such use are given in e.g. Theodora W. Greene "Protective Groups in Organic Synthesis" (Wiley-Interscience, New York 1981), and J.F.W. McOmie, "Protective Groups in Organic Chemistry" (Plenum Press, London 1973).

Salt derivatives of the compounds of formula (I) include inorganic base salts such as alkali metal salts (e.g. sodium and potassium salts) and alkaline earth metal salts (e.g. calcium and magnesium salts); ammonium salts; amino acid salts (e.g. lysine and arginine salts); organic base salts (e.g. procaine, phenylethylbenzylamine, dibenzylethylenediamine, ethanolamine, diethanolamine and N-methylglucosamine salts), and acid addition salts, e.g. formed with a mineral acid such as hydrochloric, hydrobromic, sulphuric, nitric or phosphoric acid, or with an organic acid such as formic, acetic, trifluoroacetic, methanesulphonic or p-toluenesulphonic acid. The salts may also be in the form of resinates formed with, for example, a polystyrene resin or cross-linked polystyrene divinylbenzene copolymer resin containing amino or quaternary amino groups or sulphonic acid groups, or with a resin containing carboxyl groups, e.g. a polyacrylic acid resin.

It will be appreciated that when the compound of formula (I) prepared according to the invention is ceftazidime itself, salt and ester derivatives should be non-toxic and physiologically acceptable, such as are described in UK Patent Specification No. 2025398.

Base salts of formula (I) may be formed by reacting an acid of formula (I) with an appropriate base. Thus, for example, sodium or potassium salts may be prepared using the respective 2-ethylhexanoate or hydrogen carbonate salt. Acid addition salts may be prepared by reacting a compound of formula (I) with the appropriate acid.

The reaction product may be separated from the reaction mixture, which may contain, for example, unchanged cephalosporin starting material and other substances, by a variety of processes including

solvent extraction, recrystallisation, ionophoresis, column chromatography and use of ion-exchangers (for example by chromatography on ion-exchange resins) or macroreticular resins.

Where a compound of formula (I) is obtained as a mixture of isomers, the syn isomer may be obtained by, for example, conventional methods such as crystallisation or chromatography.

A preferred embodiment of the invention relates to the preparation of ceftazidime or a protected derivative thereof, i.e., the compound of formula (I) in which $R^1$ represents an amino or protected amino group, $R^2$ represents hydrogen or an esterifying group, B represents $>$S, and the dotted line represents a ceph-3-em compound, followed if desired by removal of the protecting and/or esterifying groups, optionally in situ.

A compound of formula (II) may be prepared, for example, by acylating a compound of formula (IV)

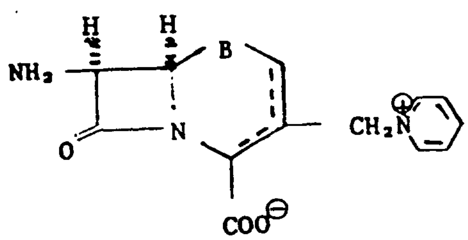

(IV)

(wherein B and the dotted line are as defined above) or a corresponding compound having at the 4-position a group $-COOR^3$, and an associated anion $A^{\ominus}$ (where $R^3$ and $A^{\ominus}$ are as defined above) or a salt e.g. an acid addition salt or N-silyl derivative thereof, with an acid of formula (V)

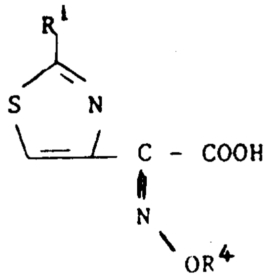

(V)

(wherein $R^1$ has the meaning defined for formula (I) and $R^4$ represents hydrogen or a hydroxyl protecting group), or an acylating agent corresponding thereto, followed by removal of the group $R^4$, when this represents a hydroxyl protecting group.

The hydroxy protecting group will preferably be one which is readily removed under mild conditions and examples of such groups include acyl groups such as t-butoxy-carbonyl, 2,2,2-trichloroethoxycarbonyl and benzyloxycarbonyl; silyl groups such as trimethylsilyl; a tetrahydropyranyl or methoxytetrahydropyranyl group; or a methoxyisopropyl group.

The acylation reaction may be effected by conventional methods, for example as described in British Patent No. 2025398.

Thus acylating agents corresponding to the acid of formula (V) which may be employed include acid halides, e.g. acid chlorides or bromides, activated esters, and symmetrical or mixed anhydrides.

Acylation may also be effected using an acid of formula (V), in which case a condensing agent such as N,N'-dicyclohexylcarbodiimide, carbonyldiimidazole or N-ethyl-5-phenylisoxazolium perchlorate, is desirably present in the reaction mixture.

Alternatively, an activated form of an acid of formula (V) for use in the acylation reaction may be formed in situ by reacting an acid of formula (V) with a solution or suspension of a halogenating agent preformed by adding a carbonyl halide, e.g. oxalyl chloride or phosgene or a phosphoryl halide, e.g. phosphorus oxychloride, to a solvent such as a halogenated hydrocarbon, e.g. methylene chloride, containing a lower acyl tertiary amide such as N,N-dimethylformamide.

The acylation reaction may conveniently be effected in a suitable solvent or mixture of solvents, such as an alcohol e.g. aqueous ethanol or aqueous industrial methylated spirit at temperatures from -50 to +50 °C, if desired in the presence of an acid binding agent.

Compounds of formula (IV) may be prepared as described in British Patent Specification No. 2,025,398 and compounds of formula (V) may be prepared as described in British Patent Specification No. 1,587,941.

The invention will now be more particularly described in the following Examples. All temperatures are in °C.

## Example 1

[6R,7R]-7-[(Z)-2-(2-methoxyprop-2-oxyimino)-2-(2-triphenylmethylaminothiazol-4-yl)acetamido]-3-(1-pyridiniummethyl)ceph-3-em-4-carboxylate

(Z)-2-Hydroxyimino-2-(2-triphenylmethylaminothiazol-4-yl)-acetic acid (2.15 g) was suspended in methylene chloride (20 ml) and 2-methoxypropene (2 ml) was added. After 25 minutes the solution was evaporated and the residue was redissolved in methylene chloride. Further 2-methoxypropene (2 ml) was added and after 20 minutes, the solution was evaporated to a gum. This gum was dissolved in methylene chloride (5 ml) and added to the Vilsmeier reagent (prepared by mixing at -20° oxalyl chloride (0.47 ml) and N,N-dimethylformamide (0.47 ml)) at -20° with stirring and under nitrogen. The solution was stirred with ice-water cooling for five minutes and then recooled to -20°. It was then added to a solution of (6R,7R)-7-amino-3-(1-pyridiniummethyl)-ceph-3-em-4-carboxylate dihydrochloride dihydrate (1.89 g) in water (3.8 ml) and industrial methylated spirits (15 ml) containing triethylamine (2.9 ml) at -10°. The resulting solution was allowed to warm to 23° over one hour and it was then partitioned between water and methylene chloride. The aqueous layer was extracted with more methylene chloride and the combined organic layers were washed with water. The organic solution was dried with magnesium sulphate and evaporated to a gum which was triturated with diethyl ether to give the title compound (2.34 g) $[\alpha]_D^{21}$ -34.8° (c 1.08, DMSO).

## Example 2

[6R,7R]-7-[(Z)-2-Hydroxyimino-2-(2-triphenylmethylaminothiazol-4-yl)acetamido]-3-(1-pyridiniummethyl)-ceph-3-em-4-carboxylate

The product of Example 1 (1.55 g) was suspended in acetone (5 ml) with stirring and concentrated hydrochloric acid (0.16 ml) in water (1.75 ml) was added. After one hour, further hydrochloric acid (0.16 ml) was added. After a further 30 minutes, the solution was partitioned between methylene chloride and aqueous sodium bicarbonate solution. The aqueous layer was extracted with more methylene chloride and the combined organic layers were washed with water, dried with magnesium sulphate and evaporated to a foam (1.01 g), $[\alpha]_D^{21}$ -31.8° (c 0.75, DMSO).

## Example 3

(6R,7R)-7-[(Z)-2-(2-t-Butoxycarbonylprop-2-oxyimino)-2-(2-triphenylmethylaminothiazol-4-yl)acetamido]-3-(1-pyridiniummethyl)ceph-3-em-4-carboxylate

The product of Example 2 (100 mg) was dissolved in N,N-dimethylformamide (2 ml) with stirring and t-butyl 2-bromo-2-methylpropionate (45 mg) was added followed by potassium carbonate (50 mg). After stirring at 23° for three hours, the title compound had been formed as demonstrated by high pressure liquid chromatographic comparison with a standard sample, whose spectroscopic characteristics resembled those of the product obtained in Example 4(a) of UK Patent No. 2025398. The product of this Example may be deprotected for example by the methods described in UK Patent No. 2025398 and UK Patent No. 2063871.

## Claims

### Claims for the following Contracting States : BE, CH, DE, FR, GB, IT, LI, NL, SE

**1.** A process for the preparation of compounds of general formula (I)

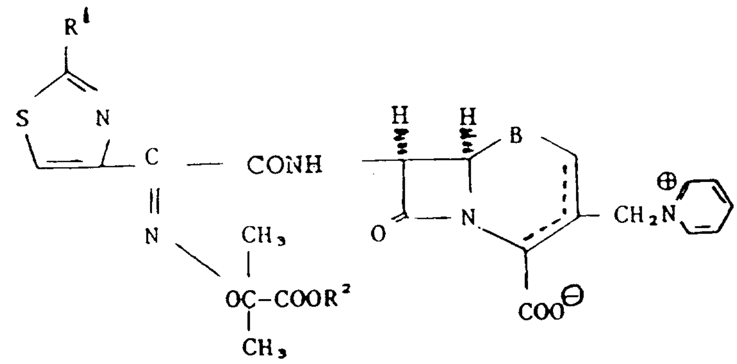

(I)

(wherein $R^1$ represents an amino or protected amino group, $R^2$ is hydrogen or a carboxyl blocking group, B is >S or >S→O and the dotted line indicates that the compound is a ceph-2-em or ceph-3-em compound) or a corresponding compound having at the 4-position a group of formula $-COOR^3$ (where $R^3$ is a carboxyl blocking group) and having an associated anion $A^{\ominus}$, and salts and esters thereof which comprises reacting a compound of formula (II)

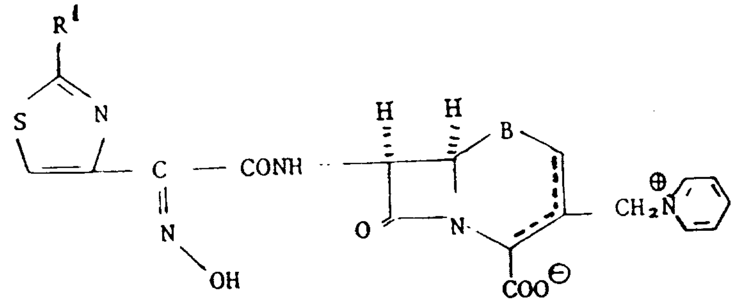

(II)

(wherein $R^1$, B and the dotted line are as defined above) or a corresponding compound having a group of formula $COOR^3$ at the 4-position (wherein $R^3$ is as defined above) together with an associated anion $A^{\ominus}$, or a salt thereof, with a compound of formula (III)

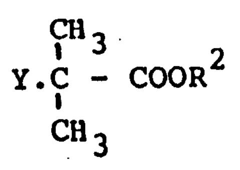

(III)

wherein Y is a leaving group and $R^2$ is as defined above in the presence of a base selected from an alkali metal carbonate, hydride, alkoxide, dialkylamide or a disilylamide.

**2.** A process as claimed in claim 1 wherein reaction is carried out in an ether, amide, ketone, nitrile, nitroalkane, sulphoxide, sulphone, hydrocarbon or ester, or a mixture of two or more thereof.

**3.** A process as claimed in claim 1 wherein reaction is carried out in a two liquid phase reaction medium in the presence of a phase transfer reagent.

**4.** A process as claimed in any of claims 1 to 3 wherein the reaction is followed, where necessary or desired, by conversion of the compound of formula (I) initially formed into a different compound of

formula (I) by means of one or more of the following reactions

(i) reduction of a compound wherein B is >S→O to a compound wherein B is >S;

(ii) conversion of a $\Delta^2$-isomer into a $\Delta^3$-isomer;

(iii) removal of any carboxyl-blocking or amino protecting groups; and

(iv) formation of a non-toxic salt or a non-toxic metabolically labile ester.

**5.** A process as claimed in any of claims 1 to 4 wherein, in the compound of formula (I) obtained, $R^1$ represents an amino group, $R^2$ represents a hydrogen atom or esterifying group, the dotted line represents a ceph-3-em compound and B is >S.

**6.** (6R,7R)-7-[(Z)-2-(2-methoxyprop-2-oxyimino)-2-(2-triphenylmethylaminothiazol-4-yl)acetamido]-3-(1-pyridiniummethyl)ceph-3-em-4-carboxylate.

**7.** (6R,7R)-7-[(Z)-2-Hydroxyimino-2-(2-triphenylmethylaminothiazol-4-yl)acetamido]-3-(1-pyridiniummethyl)-ceph-3-em-4-carboxylate.

**Claims for the following Contracting State : AT**

**1.** A process for the preparation of compounds of general formula (I)

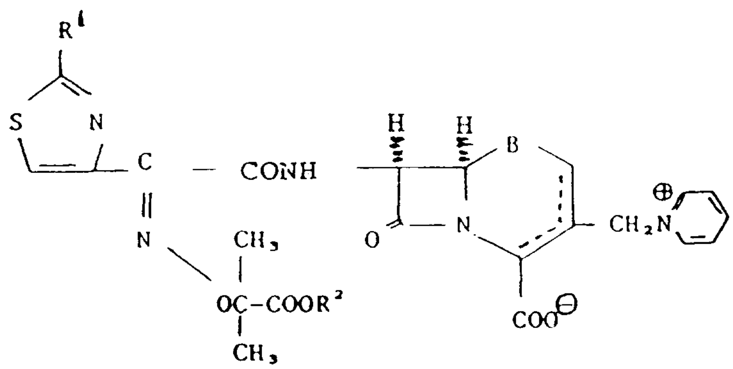

(I)

(wherein $R^1$ represents an amino or protected amino group, $R^2$ is hydrogen or a carboxyl blocking group, B is >S or >S→O and the dotted line indicates that the compound is a ceph-2-em or ceph-3-em compound) or a corresponding compound having at the 4-position a group of formula $-COOR^3$ (where $R^3$ is a carboxyl blocking group) and having an associated anion $A^{\ominus}$, and salts and esters thereof which comprises reacting a compound of formula (II)

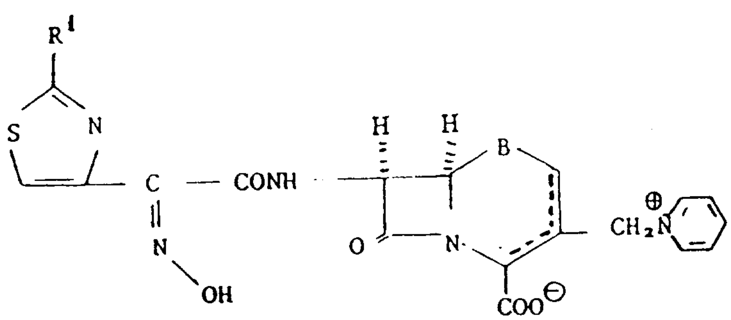

(II)

(wherein $R^1$, B and the dotted line are as defined above) or a corresponding compound having a group of formula $COOR^3$ at the 4-position (wherein $R^3$ is as defined above) together with an associated anion $A^{\ominus}$, or a salt thereof, with a compound of formula (III)

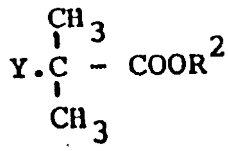

(III)

wherein Y is a leaving group and $R^2$ is as defined above in the presence of a base selected from an alkali metal carbonate, hydride, alkoxide, dialkylamide or a disilylamide.

**2.** A process as claimed in claim 1 wherein reaction is carried out in an ether, amide, ketone, nitrile, nitroalkane, sulphoxide, sulphone, hydrocarbon or ester, or a mixture of two or more thereof.

**3.** A process as claimed in claim 1 wherein reaction is carried out in a two liquid phase reaction medium in the presence of a phase transfer reagent.

**4.** A process as claimed in any of claims 1 to 3 wherein the reaction is followed, where necessary or desired, by conversion of the compound of formula (I) initially formed into a different compound of formula (I) by means of one or more of the following reactions
(i) reduction of a compound wherein B is $>S\rightarrow O$ to a compound wherein B is $>S$;
(ii) conversion of a $\Delta^2$-isomer into a $\Delta^3$-isomer;
(iii) removal of any carboxyl-blocking or amino protecting groups; and
(iv) formation of a non-toxic salt or a non-toxic metabolically labile ester.

**5.** A process as claimed in any of claims 1 to 4 wherein, in the compound of formula (I) obtained, $R^1$ represents an amino group, $R^2$ represents a hydrogen atom or esterifying group, the dotted line represents a ceph-3-em compound and B is $>S$.

**6.** A process as claimed in claim 1 wherein the compound of formula (II) is (6R,7R)-7-[(Z)-2-Hydroxyimino -2-(2-triphenyl-methylaminothiazol-4-yl)acetamido]-3-(1-pyridinium-methyl)ceph-3-em-4-carboxylate.

**7.** A process as claimed in claim 6 wherein the compound of formula (II) defined therein is prepared by the removal of the hydroxyl protecting group from (6R,7R)-7-[(Z)-2-(2-methoxyprop-2-oxyimino)-2-(2-triphenylmethylaminothiazol -4-yl)acetamido]-3-(1-pyridiniummethyl)ceph-3-em-4-carboxylate.

**Patentansprüche**
**Patentansprüche für folgende Vertragsstaaten : BE, CH, DE, FR, GB, IT, LI, NL, SE**

**1.** Verfahren zur Herstellung von Verbindungen der allgemeinen Formel (I)

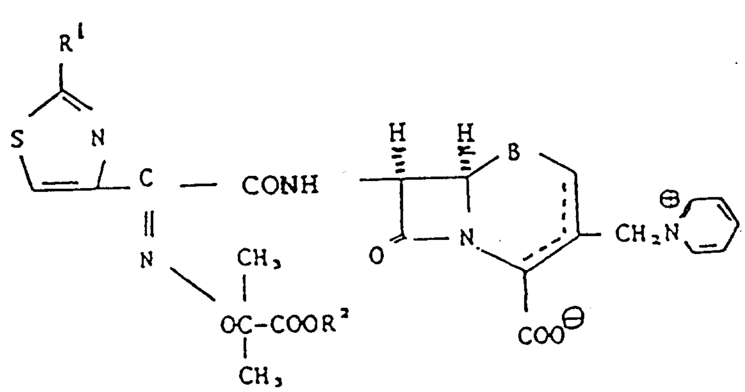

(I)

(worin $R^1$ eine Amino- oder geschützte Aminogruppe darstellt, $R^2$ Wasserstoff oder eine Carboxylblokkierungsgruppe ist, B $>S$ oder $>S\rightarrow 0$ ist und die strichlierte Linie anzeigt, daß die Verbindung eine Ceph-2-em oder Ceph-3-em Verbindung ist) oder einer entsprechenden Verbindung, die an der 4-Position eine Gruppe der Formel -$COOR^3$ (wobei $R^3$ eine Carboxylblockierungsgruppe ist) und ein

hinzugefügtes Anion $A^{\ominus}$ besitzt, sowie von Salzen und Estern davon, welches die Umsetzung einer Verbindung der Formel (II)

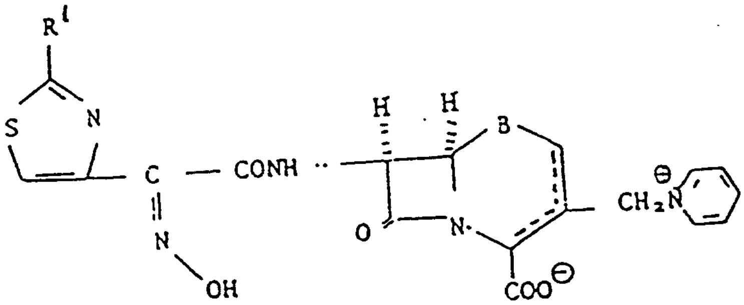

(II)

(worin $R^1$, B und die strichlierte Linie wie oben definiert sind) oder einer entsprechenden Verbindung, die eine Gruppe der Formel $COOR^3$ an der 4-Position (worin $R^3$ wie oben definiert ist) gemeinsam mit einem hinzugefügten Anion $A^{\circ}$ besitzt, oder eines Salzes davon mit einer Verbindung der Formel III umfaßt

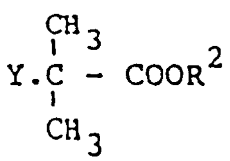

(III)

worin Y eine Austrittsgruppe und $R^2$ wie oben definiert ist, in Gegenwart einer Base, ausgewählt aus einem Alkalimetallcarbonat, Hydrid, Alkoxid, Dialkylamid oder einem Disilylamid.

**2.** Verfahren nach Anspruch 1, bei welchem die Reaktion in einem Ether, Amid, Keton, Nitril, Nitroalkan, Sulphoxid, Sulphon, Kohlenwasserstoff oder Ester oder einer Mischung von zwei oder mehreren davon ausgeführt wird.

**3.** Verfahren nach Anspruch 1, bei welchem die Reaktion in einem Zweiflüssigphasen-Reaktionsmedium in Gegenwart eines Phasenübertragungsreagens durchgeführt wird.

**4.** Verfahren nach einem der Ansprüche 1 bis 3, bei welchem auf die Reaktion, falls erforderlich oder gewünscht, eine Umwandlung der anfangs gebildeten Verbindung der Formel (I) zu einer anderen Verbindung der Formel (I) durch eine oder mehrere der folgenden Reaktionen folgt

(i) Reduktion einer Verbindung, in welcher B $>S \rightarrow O$ ist, zu einer Verbindung, worin B $>S$ ist;
(ii) Umwandlung eines $\Delta^2$-Isomers in ein $\Delta^3$-Isomer;
(iii) Entfernung jeder Carboxylblockierungs- oder Aminoschutzgruppe; und
(iv) Bildung eines nichttoxischen Salzes oder eines nichttoxischen metabolisch labilen Esters.

**5.** Verfahren nach einem der Ansprüche 1 bis 4, bei welchem in der erhaltenen Verbindung der Formel (I) $R^1$ eine Aminogruppe darstellt, $R^2$ ein Wasserstoffatom oder eine Veresterungsgruppe darstellt, die strichlierte Linie eine Ceph-3-em Verbindung darstellt und B S ist.

**6.** (6R,7R)-7-[(Z)-2-(2-Methoxyprop-2-oxyimino)-2-(2-triphenylmethylaminothiazol-4-yl)acetamido]-3-(1-pyridiniummethyl)ceph-3-em-4-carboxylat.

**7.** (6R,7R)-7-[(Z)-2-(2-Hydroxyimino)-2-(2-triphenylmethylaminothiazol-4-yl) acetamido]-3-(1-pyridiniummethyl)ceph-3-em-4-carboxylat.

**Patentansprüche für folgenden Vertragsstaat : AT**

1. Verfahren zur Herstellung von Verbindungen der allgemeinen Formel (I)

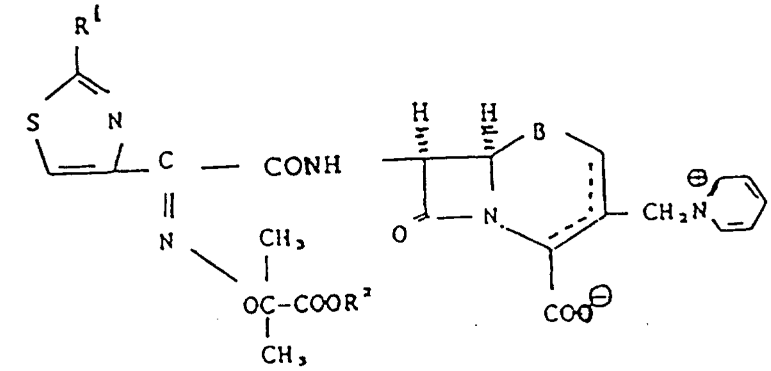

(I)

(worin $R^1$ eine Amino- oder geschützte Aminogruppe darstellt, $R^2$ Wasserstoff oder eine Carboxylblokkierungsgruppe ist, B $>S$ oder $>S \rightarrow 0$ ist und die strichlierte Linie anzeigt, daß die Verbindung eine Ceph-2-em oder Ceph-3-em Verbindung ist) oder einer entsprechenden Verbindung, die an der 4-Position eine Gruppe der Formel -$COOR^3$ (wobei $R^3$ eine Carboxylblockierungsgruppe ist) und ein hinzugefügtes Anion $A^{\ominus}$ besitzt, sowie von Salzen und Estern davon, welches die Umsetzung einer Verbindung der Formel (II)

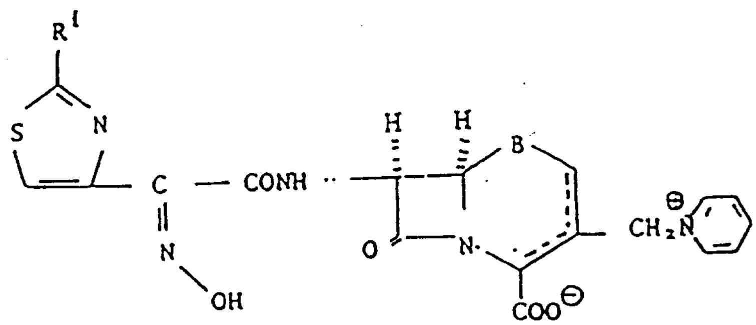

(II)

(worin $R^1$, B und die strichlierte Linie wie oben definiert sind) oder einer entsprechenden Verbindung, die eine Gruppe der Formel $COOR^3$ an der 4-Position (worin $R^3$ wie oben definiert ist) gemeinsam mit einem hinzugefügten Anion $A^{o}$ besitzt, oder eines Salzes davon mit einer Verbindung der Formel III umfaßt

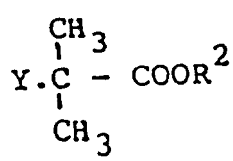

(III)

worin Y eine Austrittsgruppe und $R^2$ wie oben definiert ist, in Gegenwart einer Base, ausgewählt aus einem Alkalimetallcarbonat, Hydrid, Alkoxid, Dialkylamid oder einem Disilylamid.

2. Verfahren nach Anspruch 1, bei welchem die Reaktion in einem Ether, Amid, Keton, Nitril, Nitroalkan, Sulphoxid, Sulphon, Kohlenwasserstoff oder Ester oder einer Mischung von zwei oder mehreren davon ausgeführt wird.

3. Verfahren nach Anspruch 1, bei welchem die Reaktion in einem Zweiflüssigphasen-Reaktionsmedium in Gegenwart eines Phasenübertragungsreagens durchgeführt wird.

**4.** Verfahren nach einem der Ansprüche 1 bis 3, bei welchem auf die Reaktion, falls erforderlich oder gewünscht, eine Umwandlung der anfangs gebildeten Verbindung der Formel (I) zu einer anderen Verbindung der Formel (I) durch eine oder mehrere der folgenden Reaktionen folgt

(i) Reduktion einer Verbindung, worin B $>S\rightarrow O$ ist, zu einer Verbindung, worin B $>S$ ist;

(ii) Umwandlung eines $\Delta^2$-Isomers in ein $\Delta^3$-Isomer;

(iii) Entfernung jeder Carboxylblockierungs- oder Aminoschutzgruppe; und

(iv) Bildung eines nichttoxischeen Salzes oder eines nichttoxischen metabolisch labilen Esters.

**5.** Verfahren nach einem der Ansprüche 1 bis 4, bei welchem in der erhaltenen Verbindung der Formel (I) $R^1$ eine Aminogruppe darstellt, $R^2$ ein Wasserstoffatom oder eine Veresterungsgruppe darstellt, die strichlierte Linie eine Ceph-3-em Verbindung darstellt und B S ist.

**6.** Verfahren nach Anspruch 1, worin die Verbindung der Formel (II) (6R,7R)-7-[(Z)-2-(2-Hydroxyimino)-2-(2-triphenylmethylaminothiazol-4-yl)acetamid o]-3-(1-pyridiniummethyl)ceph-3-em-4-carboxylat ist.

**7.** Verfahren nach Anspruch 6, worin die darin definierte Verbindung der Formel (II) durch die Entfernung der Hydroxylschutzgruppe von (6R,7R)-7-[(Z)-2-(2-Methoxyprop-2-oxyimino)-2-(2-triphenylmethylaminothiazol-4-yl)acetamido]-3-(1-pyridiniummethyl)ceph-3-em-4-carboxylat hergestellt wird.

## Revendications

**Revendications pour les Etats contractants suivants : BE, CH, DE, FR, GB, IT, LI, NL, SE**

**1.** Procédé de préparation de composés de formule développée (I)

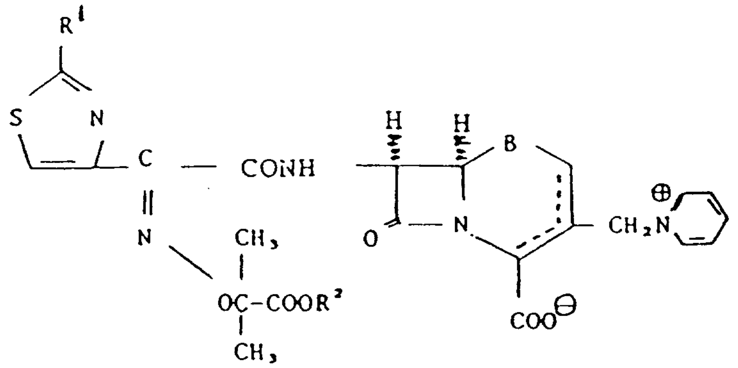

(I)

(dans laquelle $R^1$ représente un groupe amino ou amino protégée, $R^2$ est l'hydrogène ou un groupe carboxyle de blocage, B est $>S$ ou $>SO\rightarrow O$ et la ligne en tirets indique que le composé est un composé ceph-2-em ou ceph-3-em) ou un composé correspondant ayant en la position 4 un groupe de formule $-COOR^3$ ($R^3$ étant un groupe carboxyle de blocage) et un anion $A^{\ominus}$ associé, et leurs sels et esters, qui consiste à mettre un composé de formule (II)

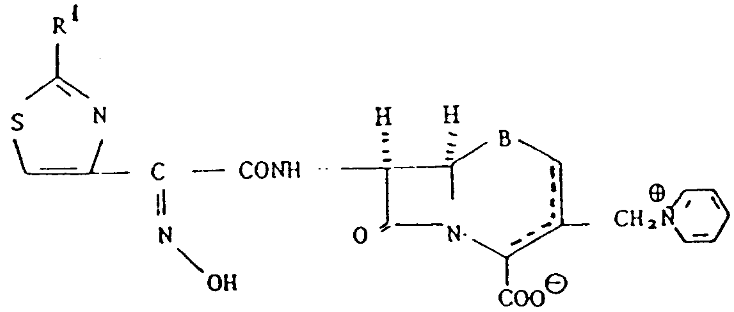

(II)

(où $R^1$, B et la ligne en tirets sont tels que définis ci-dessus) ou un composé correspondant ayant un groupe de formule $COOR^3$ en la position 4 ($R^3$ étant tel que défini ci-dessus) ensemble avec un anion

$A^{\ominus}$ associé, ou l'un de ses sels, à réagir sur un composé de formule (III)

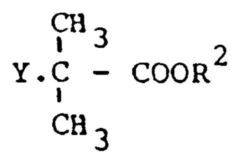

(III)

dans laquelle Y est un groupe labile et $R^2$ est tel que défini ci-dessus en la présence d'une base choisie parmi un carbonate de métal alcalin, un hydrure, un alcoolate, un dialcoylamide ou un disilylamide.

**2.** Procédé suivant la revendication 1, qui consiste à effectuer la réaction dans un étheroxyde, un amide, une cétone, un nitrile, un nitroalcane, un sulfoxide, une sulfone, un hydrocarbure ou un ester, ou un mélange de deux ou plusieurs d'entre eux.

**3.** Procédé suivant la revendication 1, qui consiste à effectuer la réaction dans un milieu réactionnel en phase liquide en la présence d'un réactif de transfert de phase.

**4.** Procédé suivant l'une quelconque des revendications 1 à 3, qui consiste à faire suivre la réaction, si nécessaire ou souhaitée, de la transformation du composé de formule (I) formé initialement en un composé différent de formule (I) au moyen de l'une ou plusieurs des réactions suivantes
(i) réduction d'un composé dans lequel B est >S→O en un composé dans lequel B est >S;
(ii) conversion d'un isomère $\Delta^2$ en un isomère $\Delta^3$
(iii) élimination de tout groupe de blocage carboxyle ou amino; et
(iv) formation d'un sel non toxique ou d'un ester métaboliquement labile non toxique.

**5.** Procédé suivant l'une quelconque des revendications 1 à 4 dans lequel, dans le composé de formule (I) obtenu, $R^1$ représente un groupe amino, $R^2$ représente un atome d'hydrogène ou un groupe estérifiant, la ligne en tirets représente un composé cèph-3-em et B est >S.

**6.** Le (6R,7R)-7-[(Z)-2-(2-méthoxyprop-2-oxyimino)-2-(2-triphénylméthyl-aminothiazol-4-yl)acétamido]-3-(1-pyridiniumméthyl)cèph-3-em-4-carboxylate.

**7.** Le (6R,7R)-7-[(Z)-2-hydroxyimino-2-(2-triphénylméthylaminothiazol-4-yl)acétamido]-3-(1-pyridinium-méthyl)cèph-3-em-4-carboxylate.

**Revendications pour l'Etat contractant suivant : AT**

**1.** Procédé de préparation de composés de formule développée (I)

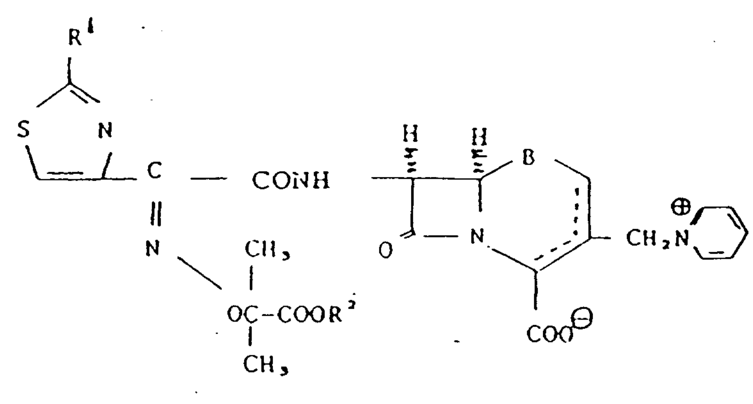

(I)

(dans laquelle $R^1$ représente un groupe amino ou amino protégée, $R^2$ est l'hydrogène ou un groupe carboxyle de blocage, B est S ou SO→O et la ligne en tirets indique que le composé est un composé

ceph-2-em ou ceph-3-em) ou un composé correspondant ayant en la position 4 un groupe de formule $-COOR^3$ ($R^3$ étant un groupe carboxyle de blocage) et ayant un anion $A^{\ominus}$ associé, et leurs sels et esters, qui consiste à mettre un composé de formule (II)

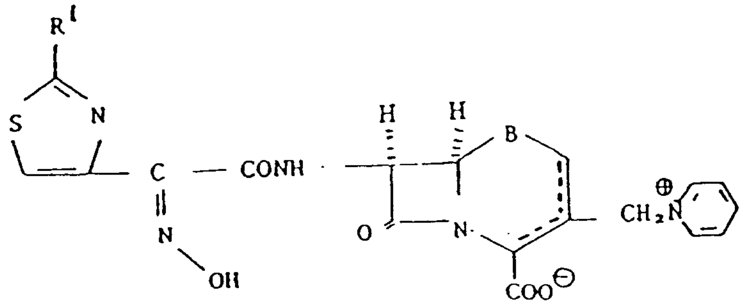

(II)

(où $R^1$, B et la ligne en tirets sont tels que définis ci-dessus) ou un composé correspondant ayant un groupe de formule $COOR^3$ en la position 4 ($R^3$ étant tel que défini ci-dessus) ensemble avec un anion $A^{\ominus}$ associé, ou l'un de ses sels, à réagir sur un composé de formule (III)

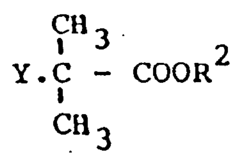

(III)

dans laquelle Y est un groupe labile et $R^2$ est tel que défini ci-dessus en la présence d'une base choisie parmi un carbonate de métal alcalin, un hydrure, un alcoolate, un dialcoylamide ou un disilylamide.

**2.** Procédé suivant la revendication 1 qui consiste à effectuer la réaction dans un étheroxyde, un amide, une cétone, un nitrile, un nitroalcane, un sulfoxide, une sulfone, un hydrocarbure ou un ester, ou un mélange de deux ou plusieurs d'entre eux.

**3.** Procédé suivant la revendication 1, qui consiste à effectuer la réaction dans un milieu réactionnel en phase liquide en la présence d'un réactif de transfert de phase.

**4.** Procédé suivant l'une quelconque des revendications 1 à 3, qui consiste à faire suivre la réaction, si nécessaire ou souhaitée, de la transformation du composé de formule (I) formé initialement en un composé différent de formule (I) au moyen de l'une ou plusieurs des réactions suivantes

(i) réduction d'un composé dans lequel B est $>S \rightarrow O$ en un composé dans lequel B est $>S$;
(ii) conversion d'un isomère $\Delta^2$ en un isomère $\Delta^3$
(iii) élimination de tout groupe de blocage carboxyle ou amino; et
(iv) formation d'un sel non toxique ou d'un ester métaboliquement labile non toxique.

**5.** Procédé suivant l'une quelconque des revendications 1 à 4 dans lequel, dans le composé de formule (I) obtenu, $R^1$ représente un groupe amino, $R^2$ représente un atome d'hydrogène ou un groupe estérifiant, la ligne en tirets représente un composé cèph-3-em et B est $>S$.

**6.** Procédé suivant la revendication 1 dans lequel le composé de formule (II) est le (6R,7R)-7-[(Z)-2-hydroxyimino-2-(2-triphényl-méthylaminothiazol-4-yl)acétamido]-3-(1-pyridinium-méthyle)cèph-3-em-4-carboxylate.

**7.** Procédé suivant la revendication 6, dans lequel le composé de formule (II) qui y est défini est préparé en éliminant le groupe hydroxyle protecteur du (6R,7R)-7-[(Z)-2-(2-méthoxyprop-2-oxyimino)-2-(2-triphénylméthylaminothiazol-4-yl)acétamido]-3-(1-pyridiniumméthyl)cèph-3-em-4-carboxylate.